(19) Europäisches Patentamt
European Patent Office
Office européen des brevets

(11) Publication number: **0 325 015 A1**

# (12) EUROPEAN PATENT APPLICATION

(21) Application number: **88300481.4**

(22) Date of filing: **21.01.88**

(51) Int. Cl.$^4$: **C07D 487/08 , //C08G18/20, (C07D487/08,241:00,241:00)**

(43) Date of publication of application: **26.07.89 Bulletin 89/30**

(84) Designated Contracting States: **BE DE GB NL**

(71) Applicant: **TEXACO DEVELOPMENT CORPORATION**
**2000 Westchester Avenue**
**White Plains New York 10650(US)**

(72) Inventor: **Zimmerman, Robert LeRoy**
**4202 Cordova**
**Austin Texas 78759(US)**

(74) Representative: **Ben-Nathan, Laurence Albert et al**
**Urquhart-Dykes & Lord 91 Wimpole Street**
**London W1M 8AH(GB)**

## (54) Anti-caking agent for triethylenediamine.

(57) A method for imparting anti-caking properties to TEDA which comprises admixing with TEDA an effective amount of an additive comprising polyoxyalkylene amines or their amides, the amines having the formula:

$$[H_2N\text{-}(\underset{X}{CH}\text{-}\underset{H}{CH}\text{-}O)_n]_rZ$$

wherein X is hydrogen, a methyl radical or an ethyl radical, Z is a hydrocarbon radical having from 2 to 5 carbon atoms forming from 2 to 4 external ether linkages; n is a number from 1 to about 40 and r is a number from 2 to 4. The invention also relates to a composition for imparting anti-caking properties to TEDA.

EP 0 325 015 A1

## ANTI-CAKING AGENT FOR TRIETHYLENEDIAMINE

Triethylenediamine (TEDA) is a well known catalyst used in the manufacture of polyurethane products. Triethylenediamine is also known as 1,4-diazabicyclo(2.2.2) octane. Many methods are known for its commercial production; for example, U.S. Patents 2,937,176; 3,166,558; 2,985,658; 2,977,364 and 2,977,363.

However, TEDA once isolated is hygroscopic. Therefore, upon storage the purified commercial product forms a hard crust at the surface which will become a thicker crust when stored for longer periods. This property makes it difficult to use since it is not a free-flowing powder once the crust is formed.

U. S. 4,345,079 discloses and claims the use of polyethylene glycols, glycol esters, glycol ethers and amino alcohols to improve this crusting problem. This patent describes the improved property as scoopability. Among the preferred additives mentioned in the '079 patent are polyethylene glycols and glycol esters.

### SUMMARY OF THE INVENTION

The invention is a method for imparting anti-caking properties to TEDA which comprises admixing with TEDA an effective amount of an additive comprising polyoxyalkylene amines or their amides, the amines having the formula:

$$[H_2N-(\underset{X}{\underset{|}{C}H}-\underset{H}{\underset{|}{C}H}-O)_n]_rZ$$

wherein X is hydrogen, a methyl radical or an ethyl radical, Z is a hydrocarbon radical having from 2 to 5 carbon atoms forming from 2 to 4 external ether linkages; n is a number from 1 to about 40 and r is a number from 2 to 4. The invention also relates to a composition for imparting anti-caking properties to TEDA.

The additive may be mixed with TEDA in any conventional manner effective to disperse the additive throughout the TEDA.

The additives preferred for this invention may be depicted by the formula:

$$[H_2N-(\underset{X}{\underset{|}{C}H}-\underset{H}{\underset{|}{C}H}-O)_n]_rZ$$

wherein X is hydrogen, a methyl radical or an ethyl radical, Z is a hydrocarbon radical having from 2 to 5 carbon atoms forming from 2 to 4 external ether linkages; n is a number from 1 to about 40 and r is a number from 2 to 4. The most preferred polyoxyalkylene polyamines are the polyoxypropylene diamines wherein X is a methyl radical, n is a number from 2 to 17, Z is a 1,2-propylene radical and r is about 2 or 3. These polyoxyalkylene polyamines can be prepared by known methods as disclosed in U. S. Patents 3.236,895 and 3,654,370.

Also useful are mono oxyalkylene polyamines. It has been found that the oxypropylene amines are useful while oxyethylene amines have not performed well.

The range of concentration of the additive by weight percent based on the TEDA is from about 0.1 to about 2.0. It is preferred to use from about 0.8 to about 1.2.

The usefulness of the invention is depicted below where TEDA was blended with the additive, then the mixture was aged at 49° C. After aging, the blends were judged to see if a crust had formed and if they were scoopable.

Table I

| | 1 | 2 | 3 | 4 | 5 | 6 | 7 | 8 | 9 | 10 |
|---|---|---|---|---|---|---|---|---|---|---|
| Triethylenediamine | 125 | 125 | 125 | 125 | 125 | 125 | 125 | 125 | 125 | 125 |
| JEFFAMINE® D-230 | - | 1.25 | - | - | - | - | - | - | - | - |
| Diacetate of JEFFAMINE D-230 | - | - | 1.25 | - | - | - | - | - | - | - |
| JEFFAMINE D-400 | - | - | - | 1.25 | - | - | - | - | - | - |
| JEFFAMINE D-2000 | - | - | - | - | 1.25 | - | - | - | - | - |
| JEFFAMINE T-403 | - | - | - | - | - | 1.25 | - | - | - | - |
| JEFFAMINE ED-600 | - | - | - | - | - | - | 1.25 | - | - | - |
| JEFFAMINE ED-900 | - | - | - | - | - | - | - | 1.25 | - | - |
| JEFFAMINE ED-2001 | - | - | - | - | - | - | - | - | 1.25 | - |
| JEFFAMINE M-1000 | - | - | - | - | - | - | - | - | - | 1.25 |
| Days aged at 49° C | 30 | 30 | 30 | 29 | 29 | 29 | 29 | 29 | 29 | 29 |
| Scoopable | no | yes | yes | yes | yes | yes | no | no | no | no |

The JEFFAMINE products that contain only propylene oxide worked well, while those containing ethylene oxide did not.

When No. 2, 3, 4, 5 and 6 were blended 1:2 with dipropylene glycol clear solutions resulted.

JEFFAMINE D-230 - a polyoxypropylene diamine of approx. 230 mol. wt.

JEFFAMINE D-400 - a polyoxypropylene diamine of approx. 400 mol. wt..

JEFFAMINE D-2000 - a polyoxypropylene diamine of approx. 2000 mol. wt.

JEFFAMINE T-403 - a polyoxypropylene triamine of approx. 400 mol. wt.

JEFFAMINE ED-600 - a polyoxyethylene-polyoxypropylene diamine of approx. 600 mol. wt.

JEFFAMINE ED-900 - a polyoxyethylene-polyoxypropylene diamine of approx. 900 mol. wt.

JEFFAMINE ED-2001 - a polyoxyethylene-polyoxypropylene diamine of approx. 2000 mol. wt.

JEFFAMINE M-1000 - a polyoxyethylene-polyoxypropylene mono amine of approximately 1000 mol. wt.

All JEFFAMINE products are supplied by Texaco Chemical Co.

## Claims

1. A composition comprising triethylenediamine with an amount of an additive effective for improving the anti-caking properties of the composition, the additive comprising compounds of the formula:

$$[H_2N\text{-}(\underset{X}{\underset{|}{C}}H\text{-}\ \underset{H}{\underset{|}{C}}H\text{-}O)_n]_rZ$$

wherein X is hydrogen, a methyl radical or an ethyl radical, Z is a hydrocarbon radical having from 2 to 5 carbon atoms forming from 2 to 4 external ether linkages; n is a number from 1 to about 40 and r is a number from 2 to 4.

2. A composition as claimed Claim 1, characterized in that the concentration of the additive by weight percent based on the triethylenediamine is from about 0.1 to about 2.0.

3. A composition as claimed in Claims 1 or 2, characterized in that X is a methyl radical, n is a number from 2 to 17, Z is a 1,2-propylene radical and r is 2 or 3.

4. A composition as claimed in any of Claims 1 to 3, characterized in that the additive is polyoxypropylene diamine of about 230 molecular weight.

5. A composition as claimed in any of Claims 1 to 3, characterized in that the additive is polyoxypropylene diamine of about 400 molecular weight.

6. A composition as claimed in any of Claims 1 to 3, characterized in that the additive is polyoxypropylene diamine of about 2000 molecular weight.

7. A composition as claimed in any of Claims 1 to 3, characterized in that the additive is polyoxypropylene triamine of about 400 molecular weight.

8. A composition as claimed in any of Claims 1 to 3, characterized in that the additive is polyoxypropylene mono amine of about 1000 molecular weight.

9. A method for improving the anti-caking properties of triethylenediamine characterized by admixing with the triethylenediamine an effective amount of an additive comprising oxyalkylene amines and their amides, the amines comprising compounds of the formula:

$$[H_2N-(\underset{\displaystyle X}{\underset{|}{C}}H-\underset{\displaystyle H}{\underset{|}{C}}H-O)_n]_rZ$$

wherein X is hydrogen, a methyl radical or an ethyl radical, Z is a hydrocarbon radical having from 2 to 5 carbon atoms forming from 2 to 4 external ether linkages; n is a number from 1 to about 40 and r is a number from 2 to 4.

European Patent Office

# EUROPEAN SEARCH REPORT

Application Number

EP 88 30 0481

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,A | DE-A-3 115 880 (AIR PRODUCTS) * Claim 1 * & US-A-4 345 079 ----- | 1 | C 07 D 487/08 // C 08 G 18/20 (C 07 D 487/08 C 07 D 241:00 C 07 D 241:00 ) |
| | | | **TECHNICAL FIELDS SEARCHED (Int. Cl.4)** |
| | | | C 07 D 487/00 C 08 G 18/00 |

The present search report has been drawn up for all claims

| Place of search | Date of completion of the search | Examiner |
|---|---|---|
| THE HAGUE | 21-09-1988 | ALFARO I. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document